# EUROPÄISCHE PATENTSCHRIFT

(11) **EP 1 003 787 B1**
(45) Veröffentlichungstag und Bekanntmachung des Hinweises auf die Patenterteilung: **21.02.2007**
(21) Anmeldenummer: 98940115.3
(22) Anmeldetag: 02.07.1998
(51) Int. Cl.: C07K 16/18, C07K 14/745, C07K 14/75, A61L 33/00, A61M 27/00, A61K 39/395

(54) **MITTEL ZUR BEHANDLUNG UND/ODER PROPHYLAXE VON MIKROZIRKULATIONSSTÖRUNGEN**
AGENT FOR THE TREATMENT AND/OR PROPHYLAXIS OF MICROCIRCULATION DISORDERS
AGENT POUR LE TRAITEMENT ET/OU LA PROPHYLAXIE DE TROUBLES DE LA MICROCIRCULATION

(30) Priorität: 10.07.1997 DE 19729591
(43) Veröffentlichungstag der Anmeldung: 31.05.2000
(73) Patentinhaber: Miltenyi Biotec GmbH, 51429 Bergisch Gladbach (DE)
(72) Erfinder: KOLL, Robert, D-85551 Kirchheim (DE); RICHTER, W.-O., D-86949 Windach-Schöffelding (DE); BIEBER, Franz, Guysborough, Nova Scotia B0H 1N0 (CA); TSCHÖPE, Wolfgang, D-85716 Unterschleissheim (DE)
(74) Vertreter: Grünecker, Kinkeldey, Stockmair & Schwanhäusser Anwaltssozietät
(86) Internationale Anmeldenummer: PCT/EP1998/004090
(87) Internationale Veröffentlichungsnummer: WO 1999/002565

(56) Entgegenhaltungen:
- EP-A- 0 225 867
- EP-A- 0 355 068
- EP-A- 0 396 188
- EP-A- 0 478 366
- EP-A- 0 714 982
- WO-A-95/31727
- WO-A-96/04304
- US-A- 5 571 708
- DATABASE WPI Week 9705 Derwent Publications Ltd., London, GB; AN 97-048322 XP002092369 & JP 08 301900 A (SHIMA KENKYUSHO KK) , 19. November 1996
- DATABASE WPI Week 8742 Derwent Publications Ltd., London, GB; AN 87-294830 XP002092370 & JP 62 205784 A (SNOW BRAND MILK PROD CO LTD & YEDA RES DEV CO LTD) , 10. September 1987
- K Y HUI ET AL: "Monoclonal Antibodies to a Synthetic Fibrin-Like Peptide Bind to Human Fibrin but Not Fibrinogen" SCIENCE, Bd. 222, September 1983, Seiten 1129-1132, XP002080953
- FERLIN G ET AL: "A NEW MONOCLONAL ANTIBODY ANTIFIBRIN: PRELIMINARY EVALUATION IN THE DETECTION OF VENOUS THROMBOSIS" INTERNATIONAL JOURNAL OF RADIATION APPLICATIONS AND INSTRUMENTATION PART B: NUCLEAR MEDICINE AND BIOLOGY, Bd. 16, Nr. 2, 1989, Seiten 163-165, XP000008197
- B KUDRYK ET AL: "Specificity of a Monoclonal Antibody for the NH2-terminal Region of Fibrin" MOLECULAR IMMUNOLOGY, Bd. 21, Nr. 1, 1984, Seiten 89-94, XP002081107
- GARGAN P E ET AL: "A MONOCLONAL ANTIBODY WHICH RECOGNISES AN EPITOPIC REGION UNIQUE TO THE INTACT FIBRIN POLYMERIC STRUCTURE" FIBRINOLYSIS, Bd. 7, Nr. 4, Juli 1993, Seiten 275-283, XP002059009

### SEQUENZPROTOKOLL

(1) ALLGEMEINE ANGABEN:
   (i) ANMELDER:
      (A) NAME: Therasorb Medizinische Systeme GmbH
      (B) STRASSE: Edisonstr.6
      (C) ORT: Unterschleissheim
      (E) LAND: Deutschland
      (F) POSTLEITZAHL: 85716
   (ii) BEZEICHNUNG DER ERFINDUNG: Mittel zur Behandlung und/ oder Prophylaxe von Mikrozirkulationsstörungen
   (iii) ANZAHL DER SEQUENZEN: 8
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (xi) SEQUENZBESCHREIBUNG: SEQ ID NO: 1:
   Gly Pro Arg Pro Xaa
   1               5
(2) ANGABEN ZU SEQ ID NO: 2:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (xi) SEQUENZBESCHREIBUNG: SEQ ID NO: 2:
   Gly Pro Arg Pro Lys
   1               5
(2) ANGABEN ZU SEQ ID NO: 3:
   (i) SEQUENZKENNZEICHEN:
      (A) LÄNGE: 4 Aminosäuren
      (B) ART: Aminosäure
      (C) STRANGFORM:
      (D) TOPOLOGIE: linear
   (ii) ART DES MOLEKÜLS: Peptid
   (xi) SEQUENZBESCHREIBUNG: SEQ ID NO: 3:
   Gly Pro Arg Xaa
   1
(2) ANGABEN ZU SEQ ID NO: 4:
   (i) SEQUENZKENNZEICHEN:
      (A) LÄNGE: 4 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: modifizierte Aminosäure
      (B) LAGE:C-terminal
      (D) SONSTIGE ANGABEN:/product= "die c-terminale Aminosäure trägt eine Säureamidgruppe als Endgruppe" /label=NH2
   (xi) SEQUENZBESCHREIBUNG: SEQ ID NO: 4:
   Gly Pro Arg Ser
   1
(2) ANGABEN ZU SEQ ID NO: 5:
   (i) SEQUENZKENNZEICHEN:
      (A) LÄNGE: 4 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: modifizierte Aminosäure
      (B) LAGE:C-terminal
      (D) SONSTIGE ANGABEN:/product= "die c-terminale Aminosäure trägt eine Säureamidgruppe als Endgruppe" /label=NH2
   (xi) SEQUENZBESCHREIBUNG: SEQ ID NO: 5:
   Gly Pro Arg Val
   1
(2) ANGABEN ZU SEQ ID NO: 6:
   (i) SEQUENZKENNZEICHEN:
      (A) LÄNGE: 3 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: modifizierte Aminosäure
      (B) LAGE:C-terminal
      (D) SONSTIGE ANGABEN:/product= "die c-terminale Aminosäure trägt eine Säureamidgruppe als Endgruppe" /label=NH2
   (xi) SEQUENZBESCHREIBUNG: SEQ ID NO: 6:
   Arg Gly Asp
   1
(2) ANGABEN ZU SEQ ID NO: 7:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: modifizierte Aminosäure
      (B) LAGE:C-terminal
      (D) SONSTIGE ANGABEN:/product= "die c-terminale Aminosäure trägt eine Säureamidgruppe als Endgruppe" /label=NH2
   (xi) SEQUENZBESCHREIBUNG: SEQ ID NO: 7:
   Glu His Ile Pro Ala
   1               5
(2) ANGABEN ZU SEQ ID NO: 8:
   (i) SEQUENZKENNZEICHEN:
      (A) LÄNGE: 9 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: modifizierte Aminosäure
      (B) LAGE:C-terminal
      (D) SONSTIGE ANGABEN:/product= "die c-terminale Aminosäure trägt eine Säureamidgruppe als Endgruppe" /label=NH2
   (xi) SEQUENZBESCHREIBUNG: SEQ ID NO: 8:
   Gly Pro Arg Pro Glu Arg His Glu Ser
   1               5

## Beschreibung

Die vorliegende Erfindung betrifft die Verwendung eines Liganden für Fibrinogen und/oder Fibrin zur Herstellung eines Mittels zur Behandlung und/oder Prophylaxe von Mikrozirkulationsstörungen und/oder zur Beeinflussung der Rheologie eines Säugers.

Die Erfindung betrifft ferner eine Adsorbersäule, die eine Matrix und einen Liganden enthält, wobei der Ligand eine Spezifität für Fibrin und/oder Fibrinogen besitzt. Weiterhin betrifft die Erfindung ein Verfahren zur Beeinflussung der Mikrozirkulation eines Säugers und eine pharmazeutische Zusammensetzung, enthaltend einen Liganden für Fibrinogen und/oder Fibrin.

Es ist seit langem bekannt, daß bestimmte Störungen und Krankheitszustände mit einem Überschuß einer bestimmten Substanz im Blut eines Patienten assoziiert sind. Zum Beispiel sind bei der Hypercholesterolämie die Spiegel des Low Density Lipoproteins (LDL) im Patientenblut aufgrund eines genetischen Defekts im LDL-Rezeptor deutlich erhöht. Die Erhöhung des LDLs kann zur Entwicklung von Arteriosklerose in den Koronararterien eines Patienten führen, was unter Umständen einen frühen Herzinfarkt oder den Tod verursachen kann.

Auch bestimmte Autoimmun- und andere Erkrankungen sind mit erhöhten Spiegeln von Substanzen im Patientenblut verbunden. Es wird zum Beispiel angenommen, daß die Symptome von Autoimmunerkrankungen, wie zum Beispiel systemischer Lupus erythematosus (SLE), rheumatoide Arthritis, idiopathische Thrombozytopenie, Myasthenia gravis und Vaskulitis durch Autoantikörper und zirkulierende Immunkomplexe im Patientenblut ausgelöst werden, die gegen die eigenen Antigene des Patienten gerichtet sind. Es wurde daher angenommen, daß die Entfernung eines großen Teils des Immunglobulins von Patienten, einschließlich Autoantikörpern und zirkulierenden Immunkomplexen (CIC), zur Verbesserung der Symptomatik führen könnte und unter Umständen sogar zu einer Heilung.

Auch für Interferon wurde diskutiert, daß es eine pathogene Substanz im Blut von Patienten sein könnte, die an Autoimmunerkrankungen, einer Allergie oder einer Abstoßung von transplantiertem Gewebe leiden. Es wurde vorgeschlagen, daß Anti-Interferon-Immunoglobuline, die an einen festen Träger gekuppelt sind, die Entfernung von Interferon aus Blut solcher Patienten bewirken könnten.

Es ist daher möglich, bestimmte Autoimmunerkrankungen zu behandeln, indem ein großer Teil der Immunoglobuline des Patienten unter Verwendung einer Säule, die mit gegen menschliches Immunoglobulin gerichteten Antikörpern beladen ist. Die Verwendung solcher Säulen bei der Behandlung von Autoimmunerkrankungen wurde zum Beispiel in den folgenden Druckschriften offenbart: Schaumann D. et al., Nieren und Hochdruckkrankheiten Nr. 9, Sept. 1994; Knöbl P. et al., Thrombosis and Haemostasis, 74 (4), 1035-1038 (1995); Tribl B. et al., Ann. Hematology 71 (1995); Richter W.-O. et al., ASAIO Journal Vol. 41, No. 1, Suppl. P. 2 (1995); Schlee H. et al., Wiener Klinische Wochenschrift 108, Suppl. 1, 27 (1996); Dörffel et al., Zeitschrift für Kardiologie, Band 85, Suppl. 2, Abstr. 667 (1996).

Bei einer Transplantation ergibt sich ebenfalls ein Bedarf, bestimmte Substanzen aus dem Blut des Patienten zu entfernen. Im allgemeinen muß das transplantierte Organ immunologisch mit dem Empfänger übereinstimmen, um eine hyperakute Abstoßung des Donororgans zu verhindern. Wenn ein Donororgan transplantiert wird, gegen das der Empfänger Antikörper gebildet hat oder bildet, tritt eine Abstoßung des Donororgans kurz nach der Transplantation auf. Eine solche Reaktion tritt auf, wenn das eigene Immunsystem des Empfängers das transplantierte Organ innerhalb von Minuten bis typischerweise innerhalb von 48 Stunden nach der Transplantation attackiert und zerstört. Selbst wenn der Empfänger eine immunosuppressive Therapie erhält, kann diese schnelle Abstoßung nicht verhindert werden.

Es wurden daher Verfahren entwickelt, mit denen Anti-A/Anti-B-Antikörper aus dem Blut des Empfängers entfernt werden, wobei eine extrakorporale Perfusion des Plasmas des Empfängers über synthetische A/B-Blutgruppenantigene verwendet wurde, die kovalent an Silica gebunden waren.

Weiterhin gibt es eine ganze Reihe von Erkrankungen, denen eine Verschlechterung der Mikrozirkulation gemeinsam ist. Diese Veränderung kann primär die auslösende Ursache der Krankheit sein oder sie kann auf die Erkrankung folgen. In jedem Fall kann sie wesentlich zum klinischen Bild beitragen.

Die Ursache der verminderten Mikrozirkulation kann vom Gefäßsystem ausgehen, indem zum Beispiel entzündliche oder metabolische Veränderungen den Gefäßdurchmesser der Arteriolen, Kapillaren und Venolen reduzieren und damit die Mikrozirkulation beeinträchtigen. Ebenso kann die Blutzusammensetzung die Mikrozirkulation beeinflussen. Entscheidend sind hierbei die Viskosität des Plasmas und die Verformbarkeit der Erythrozyten. Auch bei Störungen der Makrozirkulation spielt die Blutzusammensetzung eine Rolle, entscheidend sind die Vollblutsviskosität und die Erythrozytenaggregation.

Die Plasmaviskosität hängt von der Konzentration verschiedener Makromoleküle ab. Zum Beispiel beeinflussen Fibrinogen, IgM, α₂-Makroglobulin, aber gering auch Chylomikronen, VLDL und LDL die Plasmaviskosität konzentrationsabhängig.

Die nachfolgende Übersicht zeigt die verschiedenen komplexen Bestandteile, die die Rheologie des Blutes und damit die Mikrozirkulation beeinflussen:

Abgesehen von Herzinfarkt, koronarem Herztod oder Schlaganfall existieren eine Reihe anderer Erkrankungen, die mit Mikrozirkulationsstörungen einhergehen. Dazu gehört zum Beispiel der Typ-II-Diabetes. Die Patientengruppe der Diabetiker ist nicht zuletzt wegen der hohen Inzidenz und Prävalenz von Diabetes von erheblicher Bedeutung. Derzeit gibt es in der BRD etwa 4 Millionen Diabetiker. Etwa 15 bis 25 % haben oder entwickeln im Verlauf der Erkrankung Komplikationen, welche auf Mikrozirkulationsstörungen zurückzuführen sind.

Hierzu gehören zum Beispiel der diabetische Fuß, die Retinopathie, Polyneuropathien und beeinträchtigte Nierenfunktion. Die Bedeutung zum Beispiel des diabetischen Fußes läßt sich bereits aus der Tatsache ablesen, daß es in den USA Kliniken gibt, die sich ausschließlich auf die Therapie des diabetischen Fußes spezialisiert haben.

Weitere Krankheiten, die mit Mikrozirkulationsstörungen einhergehen, sind insbesondere die arterielle Verschlußkrankheit, Hörsturz und Sepsis. Die folgende Auflistung zeigt Krankheiten, die mit Mikrozirkulationsströungen einhergehen können:

| | |
|---|---|
| ZNS | Schlaganfall |
| | TIA (Transient Ischemic Attack) |
| | PRIND (Prolonged Reversible Ischemic Neurological Deficit) |
| | Chronische vaskuläre Erkrankungen des ZNS |
| | Chronische intracranielle Durchblutungsstörungen |
| | Chronische extracranielle Durchblutungsstörungen |
| | Zerebrovaskuläre Durchblutungsstörungen |
| | Demenz |
| | Alzheimer Krankheit |
| | Schwerer zentraler Schwindel |
| Auge | Chronische Durchblutungsstörung |
| | Akuter Gefäßverschluß |
| Ohr | Hörsturz |
| | Innenohr bedingter Schwindel |
| | Morbus Menière |
| Lunge | Primäre Pulmonale Hypertonie |
| | Venoocclusive Erkrankungen der Lunge |
| | Thrombotische primäre pulmonale Hypertonie |
| | Thromboembolische Erkrankungen der großen Gefäße |
| Herz | Transplantationsvaskulopathien |
| | Akuter Myokardinfarkt |
| | Instabile Angina pectoris |
| | Small vessel disease des Herzens |
| | Nicht operable schwere koronare Herzkrankheit |
| | Kardiomyopathien |
| Abdomen | Angina abdominalis |
| Nieren | Vaskulopathien der Nieren |
| | Glomerulonephritiden |
| | Chronische Niereninsuffizienz |

Periphere arterielle Verschlußkrankheiten
Akute Gefäßverschlüsse
Vaskulitiden
Septischer Schock
Disseminierte intravaskuläre Coagulation (DIC) anderer Genese, z.B. bei Tumorerkrankungen
Diabetes Typ I + II
Diabetische Retinopathie
Diabetische Neuropathie
Diabetische Nephropathie

Die Behandlungsmöglichkeiten für die oben genannten Krankheiten, insbesondere für die dabei beteiligten Mikrozirkulationsstörungen, sind bis jetzt beschränkt.

Beispielsweise wurde die diabetische Gangrän bislang rein symptomatisch behandelt (Bettruhe, gute Blutzuckereinstellung, supportive Therapie). Venöse Thromboembotien werden mit Heparin oder Fibrinolysetherapie behandelt Bein Schlaganfall kann z.B. Aspirin eingesetzt werden, das über die Hemmung der Aggregation von Blutplättchen wirkt. Der Schock wird beispielsweise mit adrenergen Mitteln, z.B. Epinephrin behandelt. Allen diesen Mitteln ist es jedoch gemeinsam, daß sie weder geeignet sind zu einer wirklich effektiven Vorbeugung noch daß sie zufriedenstellende Ergebnisse bei der Behandlung zeigen.

Es war daher eine Aufgabe der vorliegenden Erfindung, eine wirksame Möglichkeit zu schaffen, mit der Mikrozirkulationsstörungen und die Rheologie des Blutes behandelt und beeinflußt werden können. Es ist eine weitere Aufgabe der vorliegenden Erfindung, eine Adsorbersäule anzugeben, die für eine Beeinflussung der Mikrozirkulation eines Säugers verwendet werden kann. Weiterhin ist es eine Aufgabe gemäß der vorliegenden Erfindung, ein Verfahren zur Beeinflussung der Mikrozirkulation eines Säugers anzugeben. Schließlich ist es eine Aufgabe der vorliegenden Erfindung, eine pharmazeutische Zusammensetzung anzugeben, die geeignet ist zur Behandlung und/oder Prophylaxe von Mikrozirkulationsstörungen.

Diese Aufgaben werden durch die in den unabhängigen Ansprüchen genannten Gegenstände gelöst Vorteilhafte Weiterbildungen sind in den Unteransprüchen angegeben.

Gemäß dem Anspruch 1 der vorliegenden Erfindung wird ein Ligand für Fibrinogen und/oder Fibrin zur Herstellung eines Mittels zur in-vitro Behandlung und/oder Prophylaxe von Mikrozirkulationsstörungen und/oder zur Beeinflussung der Rheologie eines Säugers verwendet.

Ligand bedeutet In diesem Zusammenhang eine Substanz, die spezifisch an Fibrin und/oder Fibrinogen bindet, vorzugsweise ist die Bindung reversibel.

Vorzugsweise handelt es sich bei dem Liganden um ein Peptid, wobei dieses wiederum vorzugsweise 3 bis 10 Aminosäuren aufweist. Besonders bevorzugt enthält das Peptid die folgende Aminosäuresequenz:

Gly-Pro-Arg-Pro-X,

wobei X eine beliebige Aminosäure wie z.B. Lysin oder poly-Lysin oder ein Spacer sein kann.

Als Spacer kommen beispielsweise ε-Aminocapronsäure oder 6-C-Moleküle in Betracht.

Als besonders geeignet hat sich die folgende Aminosäuresequenz für das Peptid erwiesen:

Gly-Pro-Arg-Pro-Lys.

Weitere geeignete Sequenzen sind:
Gly-Pro-Arg-X
Gly-Pro-Arg-Ser-NH₂
Gly-Pro-Arg-Val-NH₂
Arg-Gly-Asp-NH₂
Glu-His-Ile-Pro-Ala-NH₂
Gly-Pro-Arg-Pro-Glu-Arg-His-Glu-Ser-NH₂

In einer weiteren Ausführungsform der vorliegenden Erfindung kann der Ligand ein Antikörper sein. Er kann ausgewählt werden aus polyklonalen und monoklonalen Anti-Fibrinogen-Antikörpern und Anti-Fibrin-Antikörpern.

In einer weiteren bevorzugten Ausführungsform ist der Säuger ein Mensch.

Weiterhin kann der Ligand in dem Mittel an eine feste Matrix gebunden sein, wobei die Matrix aus Glas, Kohlenhydrate, Polymethacrylate und Polyamiden ausgewählt sein kann.

In einer besonders bevorzugten Ausführungsform ist die Matrix Sepharose.

Die Matrix kann aus Kügelchen, Fasern und/oder eine Membran bestehen.

Die Krankheiten, die mit Mikrozirkulationsstörungen einhergehen, können zum Beispiel Diabetes, Rhetinopathie, Polyneuropathie, Apoplex, Hörsturz, Sepsis, arterielle Verschlußkrankheiten und/oder eine beeinträchtigte Nierenfunktion sein.

Weiterhin ist die vorliegende Erfindung auf eine Adsorbersäule gerichtet, die eine Matrix und einen Liganden enthält, wobei der Ligand eine Spezifität für Fibrin und/oder Fibrinogen besitzt. Vorzugsweise ist der Ligand das Peptid mit der Aminosäuresequenz

Gly-Pro-Arg-Pro-X,

wobei X jede beliebige Aminosäure oder ein Spacer sein kann, und besonders bevorzugt ein Peptid mit der Aminosäuresequenz

Gly-Pro-Arg-Pro-Lys.

Die Matrix in der Adsorbersäule ist vorzugsweise Sepharose. Die Herstellung der Adsorbersäule kann beispielsweise gemäß WO 95/31727 erfolgen.

Weiterhin ist die vorliegenden Erfindung auf ein Verfahren zur Beeinflussung der Mikrozirkulation eines Säugers gerichtet, wobei in vitro Blut oder Plasma des Säugers in die oben beschriebene Säule geführt wird. Als bevorzugtes Verfahren wird ein Aphereseverfahren durchgeführt, bei dem in einem Kreislauf dem Patienten Blut entnommen, dieses in Blutzellen und Plasma aufgetrennt und über die Adsorbersäule geleitet und anschließend dem Patienten wieder zurückgeführt wird.

Durch die vorliegende Erfindung wird es möglich, Blut eines Patienten mit Mikrozirkuladonsstörungen über eine Säule zu führen, die beispielsweise als Matrix Sepharose und als Liganden das oben genannte Peptid enthält und dadurch Fibrinogen und/oder Fibrin aus dem Blut zu entfernen. Das Blut kann anschließend zum Patienten zurückgeleitet werden, woraufhin sich dessen Fibrinogen und/oder Fibringehalt im Blut deutlich verringert. Es wurde gefunden, daß ein verringerter Fibrinogen- und/oder Fibringehalt im Blut direkt mit einer Verringerung von Mikrozirkutationsstörungen und damit einer Verbesserung der jeweiligen Krankheitssymptome einher geht.

Eine hemorheologisch wirksame Absenkung der Gesamtfibrinogenmenge im Blut bedeutet eine deutliche Verbesserung der Situation und trägt erheblich zur Behandlung und/oder Prophylaxe von Mikrozirkulationsstörungen bei. Dies bedeutet in der Regel einen Zielwert von 50 bis 100 mg/dl des Patientenbluts oder eine zu entfernende Fibrinogenmenge von 12 bis 13,5 g pro Patient im Durchschnitt. Daher sollte ein Absorber im Doppelsäulenprinzip etwa 2,5 bis 3 g Bindungskapazität aufweisen.

Die oben genannten Werte ergeben sich aus durchschnittlichen hohen Fibrinogenkonzentrationen von ca. 500 mg/dl und einem angenommenen Plasmavolumen von 31, damit errechnet sich eine Gesamtfibrinogenmenge von ca. 15 g.

Gemäß der vorliegenden Erfindung werden eine oder zwei Adsorbersäulen eingesetzt, mit welchen die oben genannte Absenkung erzielt werden kann.

Wegen der zu erwartenden hohen Mengen des zu absorbierenden Fibrinogens wird vorzugsweise das Doppelsaulenprinzip angewendet, um
- die Größe des Absorbers zu limitieren,
- die Kosten des Adsorberrnaterfais zu senken, und
- dadurch die zu behandelnde Plasmamenge unbegrenzt ist.

Durch den direkt mit dem Patienten verbundenen Kreislauf und der Anwendung des Doppeladsorberprinzips mit abwechselnder Beladung und Regeneration der Adsorber kann die Plasmamenge desorbiert werden, welche zur gewünschten Fibrinogenabsenkung führt. Diese Plasmabeladungsmenge sollte in der Regel nicht mehr als die 1,5- bis 2-fache Plasmamenge des Patienten darstellen.

Vorzugsweise wird eine Adsorbergröße von weniger als 200 ml verwendet.

Als Adsorbermaterial kommen die oben genannten Materialien in Frage, wobei besonders Sepharose und Membranen bevorzugt werden.

An den Liganden ist die Anforderung zu stellen, daß er eine hohe Affinität zu Fibrinogen aufweist und zu einer maximalen unspezifischen Absenkung von 10 bis 20 % pro Sitzung von Gerinnungsfaktoren, IgG, IgA, Albumin, Enzymen und Hormonen führt, wobei eine gleichzeitige Absorption von IgM, Makroglobulin, VLDL und LDL ebenfalls günstig ist. Diese liegt jedoch immer deutlich unter der prozentualen Fibrinogenabsenkung wegen des dadurch zu erwartenden günstigen rheologischen Effektes.

Eine durch Substitutionslösungen bewirkte geringe Hämodilution wirkt sich ebenfalls günstig auf die hämorheologischen Parameter aus.

Das Peptid mit der Aminosäuresequenz

Gly-Pro-Arg-Pro-Lys

weist eine sehr hohe Spezifität für Fibrinogen und/oder Fibrin auf.

Weitere Anforderungen an eine Adsorbersäule sind, daß es sich um ein Material handeln sollte, das einfach sterilisierbar sein sollte.

Im folgenden soll der Gegenstand der vorliegenden Erfindung im Hinblick auf die folgenden Beispiele im einzelnen beschrieben werden.

### BEISPIELE

### Beispiel 1 und 2

Das Pentapeptid Gly-Pro-Arg-Pro-Lys wurde als Trifluorazetat synthetisiert und an Cyanbromid-aktive Sepharose CL-4B gekoppelt. Die Spezifität der gekoppelten Sepharose wurde mittels SDS-Gelelektrophorese getestet. Das in den Säulen retendierte und anschließend eluierte Material und die Fibrinogenstandardpräparation wiesen identische Banden auf.

Mit der Peptid-gekoppelten Sepharose wurden Adsorptionssäulen hergestellt. Pro Säule wurden 3 g Sepharose (Naßgewicht) eingesetzt. Die Säulen wurden zuerst mit PBS und dann mit isotonischer Kochsalzlösung vorgespült und dann mit heparinisiertem Plasma (40 ml) beladen. Nach der Säule wurde das Plasma in Fraktionen von je 3 ml aufgefangen und in diesen Proben die Messung der Konzentration verschiedener Plasmakomponenten und der Plasmaviskosität vorgenommen. Anschließend wurden die Säulen mit Glycin-HCl-Puffer (pH 2,8) beladen und dadurch das gebundene Fibrinogen eluiert. Nach Beladung mit PBS und isotonischer Kochsalzlösung konnten die Säulen erneut beladen werden.

### Messmethoden:

Plasmaviskosität wurde bei 37°C mit einem Contraves 30 low shear Rotationsviskosimeter gemessen.

Fibrinogen und Immunoglobuline wurden mit einem Behring Laser Nephelometer immunnephelometrisch gemessen. Cholesterin und Triglyceride wurden enzymatisch bestimmt (Epos Autoanalyzer, Eppendorf, mit Reagenzien von Boehringer).

### Beispiel1

Die folgende Tabelle zeigt den Einfluß des Fibrinogenadsorbers auf die Plasmakonzentration von Fibrinogen und Cholesterin sowie den daraus resultierenden Effekt auf die Plasmaviskosität (n=7)

**TABELLE 1**

| Fraktion | Fibrinogen (g/L) | Cholesterin (mmol/L) | Plasmaviskosität (mPas) |
|---|---|---|---|
| Plasma | 3,31 ± 0,20 | 6,40 ± 0,23 | 1,27 ± 0,02 |
| 1 | 0,94 ± 0,16 | 6,23 ± 0,17 | 1,17 ± 0,01 |
| 2 | 1,27 ± 0,17 | 6,29 ± 0.17 | 1,17 ± 0,01 |
| 3 | 1,49 ± 0,17 | 6,31 ± 0.16 | 1,18 ± 0,01 |
| 4 | 1,60 ± 0,15 | 6,32 ± 0,19 | 1,19 ± 0,01 |
| 5 | 1,81 ± 0,17 | 6,32 ± 0,18 | 1,20 ± 0,02 |
| 6 | 1,87 ± 0,16 | 6,29 ± 0,19 | 1,20 ± 0,02 |

Die folgende Tabelle zeigt den Einfluß des Fibrinogenadsorbers auf die Plasmakonzentration von Fibrinogen und Triglyzeriden sowie den daraus resultierenden Effekt auf die Plasmaviskosität (n=7).

**TABELLE 2**

| Fraktion | Fibrinogen (g/L) | Cholesterin (mmol/L) | Plasmaviskosität (mPas) |
|---|---|---|---|
| Plasma | 4,29 ± 0,79 | 19,13 ± 7,04 | 1,42 ± 0,06 |
| 1 | 1,62 ± 0,70 | 16,28 ± 5,15 | 1,03 ± 0,05 |
| 2 | 1,90 ± 0,86 | 17,41 ± 5,86 | 1,22 ± 0,04 |
| 3 | 2,26 ± 0,92 | 17,54 ± 5,93 | 1,32 ± 0,05 |
| 4 | 2,52 ± 0,92 | 17,83 ± 5,97 | 1,34 ± 0,05 |
| 5 | 2,69 ± 0,90 | 18,12 ± 6,01 | 1,35 ± 0,05 |
| 6 | 2,85 ± 0,92 | 16,52 ± 5,15 | 1,33 ± 0,05 |

In beiden Versuchen korrelierte die Fibrinogenabsenkung hochsignifikant mit der Plasmaviskosität (Paired sample t test).

### Beispiel 2

In gleicher Weise hergestellte Säulen, welche statt des Pentapeptids einen polyklonalen anti-human-Immunglobulin-Antikörper vom Schaf gekoppelt an Sepharose CL-4B aufweisen (spezifische Bindung von humanen IgG- alle 4 Subklassen -, IgM, IgA, lmmunkomplexe, Bruchstücke von Immunglobulinen) zeigten ebenfalls einen Einfluß auf die Plasmaviskosität, der jedoch deutlich geringer war als mit dem Fibrinogenadsorber.

Die folgende Tabelle faßt die Ergebnisse zusammen (n=7).

**TABELLE 3**

| Einfluß eines lmmunglobulinadsorbers auf die Plasmakonzentration von Fibrinogen, Cholesterin, IgG, IgA, IgM sowie der daraus resultierende Effekt auf die Plasmaviskosität | | | | | | |
|---|---|---|---|---|---|---|
| Fraktion | Fibrinogen (g/L) | Cholesterin (g/L) | IgG (g/L) | IgA (g/L) | IgM (g/L) | Plasma-viskosität (mPas) |
| Plasma | 3,21 ± 0,20 | 6,40 ± 0,23 | 11,80 ± 0,43 | 2,96 ± 0.22 | 1,89 ± 0,25 | 1,27 ± 0,02 |
| 1 | 3,10 ± 0,20 | 6,25 ± 0,22 | 7,72 ± 0,73 | 2,77 ± 0,30 | 1,71 ± 0,27 | 1,21 ± 0,01 |
| 2 | 3,15 ± 0,21 | 6,30 ± 0,16 | 9,97 ± 0,57 | 2,87 ± 0,24 | 1,75 ± 0,28 | 1,24 ± 0,01 |
| 3 | 3,13 ± 0,21 | 6,28 ± 0,18 | 10,75 ± 0,51 | 2,88 ± 0,24 | 1,80 ± 0,29 | 1,25 ± 0,02 |
| 4 | 3,11 ± 0,20 | 6,25 ± 0,17 | 10,99 ± 0,45 | 2,83 ± 0,22 | 1,79 ± 0,29 | 1,24 ± 0,02 |
| 5 | 3,10 ± 0,20 | 6,30 ± 0,18 | 11,36 ± 0,45 | 2,85 ± 0,22 | 1,81 ± 0,29 | 1,25 ± 0,02 |
| 6 | 3,10 ± 0,19 | 6,17 ± 0,21 | 11,42+0,44 | 2,86 ± 0,20 | 1,81 ± 0,30 | 1,25 ± 0,02 |

Eine signifikante Veränderung in der Plasmaviskosität wird nur in Fraktion 1 und 2 gesehen und korrespondiert mit den niedrigsten IgG-Werten.

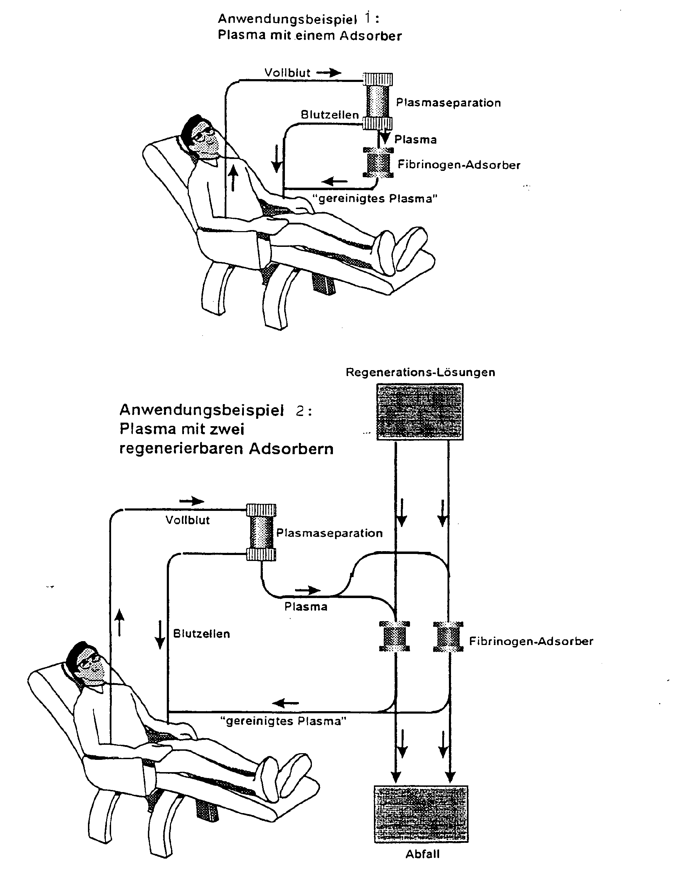

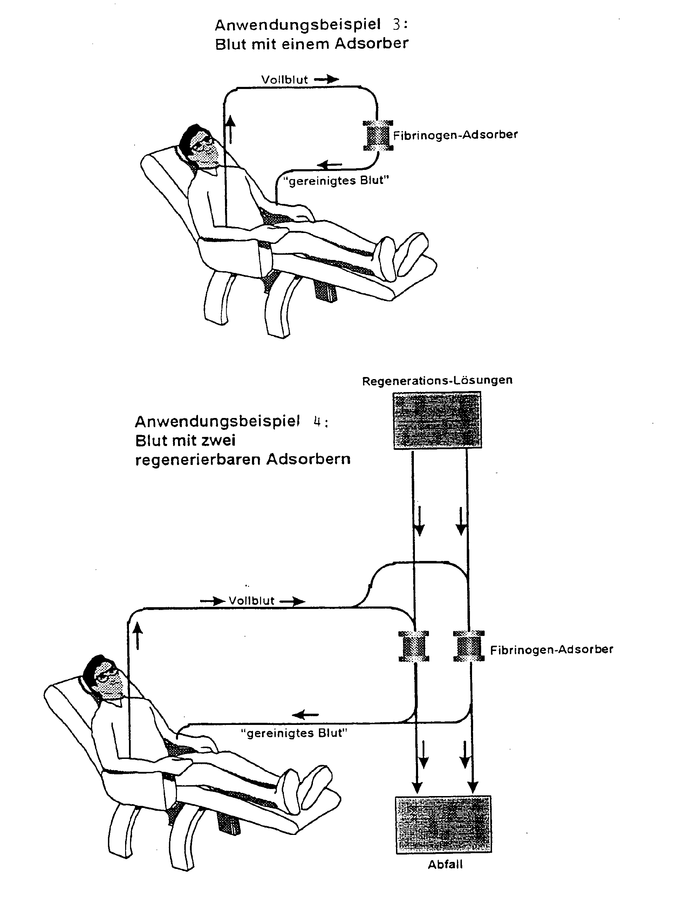

## Patentansprüche

1. Verwendung eines Liganden für Fibrinogen und/oder Fibrin zur Herstelllung eines Mittels zur *in vitro*-Behandlung und/oder Prophylaxe von Mikrozirkulationsstörungen und/oder zur Beeinflussung der Rheologie eines Säugers.

2. Verwendung nach Anspruch 1, **dadurch gekennzeichnet, daß** der Ligand ein Peptid ist, vorzugsweise mit 3 bis 10 Aminosäuren.

3. Verwendung nach Anspruch 2, **dadurch gekennzeichnet, daß** das Peptid die folgende Aminosäuresequenz enthält:
Gly-Pro-Arg-Pro-x
wobei x jede beliebige Aminosäure oder ein Spacer sein kann.

4. Verwendung nach Anspruch 3, wobei das Peptid die folgende Aminosäuresequenz aufweist:
Gly-Pro-Arg-Pro-Lys.

5. Verwendung nach Anspruch 1, **dadurch gekennzeichnet, daß** der Ligand ein Antikörper ist.

6. Verwendung nach einem der Ansprüche 1 bis 5, **dadurch gekennzeichnet, daß** der Säuger ein Mensch ist.

7. Verwendung nach einem der Ansprüche 1 bis 6, **dadurch gekennzeichnet, daß** der Ligand ausgewählt wird aus polyklonalen und monoklonalen Anti-Fibrinogen-Antikörpern und Anti-Fibrin-Antikörpern.

8. Verwendung nach einem der Ansprüche 1 bis 7, **dadurch gekennzeichnet, daß** der Ligand in dem Mittel an eine feste Matrix gebunden ist.

9. Verwendung nach Anspruch 8, **dadurch gekennzeichnet, daß** die Matrix ausgewählt wird aus Glas, Kohlenhydraten, Polymethacrylaten und Polyamiden.

10. Verwendung nach Anspruch 9, **dadurch gekennzeichnet, daß** die Matrix Sepharose ist.

11. Verwendung nach einem der Ansprüche 8 bis 10, **dadurch gekennzeichnet, daß** die Matrix aus Kügelchen, Fasern und/oder einer Membran besteht.

12. Verwendung nach einem oder mehreren der vorstehenden Ansprüche, **dadurch gekennzeichnet, daß** die Mikrozirkulationsstörung im Zusammenhang mit Diabetes, Retinopathie, Polyneuropathie, Apoplex, Hörsturz, Sepsis, arteriellen Verschlußkrankheiten und/oder beeinträchtigter Nierenfunktion auftritt.

13. Adsorbersäule, enthaltend eine Matrix und einen Liganden, wobei der Ligand eine Spezifität für Fibrin und/oder Fibrinogen besitzt.

14. Adsorbersäule nach Anspruch 13, wobei der Ligand das Peptid wie in einem der Ansprüche 3 oder 4 angegeben ist.

15. Adsorbersäule nach Anspruch 13 oder 14, wobei die Matrix Sepharose ist.

16. Verfahren zur Beeinflussung der Mikrozirkulation eines Säugers, wobei in vitro Blut des Säugers über die Säule gemäß Anspruch 13, 14 oder 15 geführt wird.

17. Verfahren nach Anspruch 16, **dadurch gekennzeichnet, daß** es als Aphereseverfahren im Plasma oder Vollblut durchgeführt wird.

## Claims

1. Use of a ligand for fibrinogen and/or fibrin for the preparation of an agent for *in vitro* treatment and/or prophylaxis of microcirculation disorders and/or influencing the rheology of a mammal.

2. Use according to Claim 1, **characterised in that** the ligand is a peptide, preferably with 3 to 10 amino acids.

3. Use according to Claim 2, **characterised in that** the peptide comprises the following amino acid sequence:
Gly-Pro-Arg-Pro-x,
wherein x can be any arbitrary amino acid or a spacer.

4. Use according to Claim 3, wherein the peptide has the following amino acid sequence:
Gly-Pro-Arg-Pro-Lys.

5. Use according to Claim 1, **characterised in that** the ligand is an antibody.

6. Use according to any one of Claims 1 to 5, **characterised in that** the mammal is a human.

7. Use according to any one of Claims 1 to 6, **characterised in that** the ligand is selected from polyclonal and monoclonal anti-fibrinogen antibodies and anti-fibrin antibodies.

8. Use according to any one of Claims 1 to 7, **characterised in that** the ligand in the agent is bound to a solid matrix.

9. Use according to Claim 8, **characterised in that** the matrix is selected from glass, carbohydrates, polymethacrylates and polyamides.

10. Use according to Claim 9, **characterised in that** the matrix is sepharose.

11. Use according to any one of Claims 8 to 10, **characterised in that** the matrix is composed of beads, fibres and/or a membrane.

12. Use according to one or more of the previous Claims, **characterised in that** the microcirculation disorder occurs in association with diabetes, retinopathy, polyneuropathy, cerebrovascular accident, apoplectiform deafness, septicaemia, arterial occlusive diseases and/or impaired renal function.

13. Adsorption column comprising a matrix and a ligand, wherein the ligand possesses specificity for fibrin and/or fibrinogen.

14. Adsorption column according to Claim 13, wherein the ligand is the peptide as defined in one of Claims 3 or 4.

15. Adsorption column according to Claim 13 or 14, wherein the matrix is sepharose.

16. Method for influencing the microcirculation of a mammal, wherein blood of the mammal is passed in vitro through the column according to Claim 13, 14 or 15.

17. Method according to Claim 16, **characterised in that** it is carried out as an apheresis procedure in plasma or whole blood.

## Revendications

1. Utilisation d'un ligand du fibrinogène et/ou de la fibrine pour préparer un agent destiné au traitement et/ou à la prévention de troubles de la microcirculation *in vitro* et/ou à agir sur la rhéologie d'un mammifère.

2. Utilisation selon la revendication 1, **caractérisée en ce que** le ligand est un peptide, comportant de préférence de 3 à 10 acides aminés.

3. Utilisation selon la revendication 2, **caractérisée en ce que** le peptide contient la séquence d'acides aminés suivante :
Gly-Pro-Arg-Pro-x
où x peut être un acide aminé quelconque ou un segment espaceur.

4. Utilisation selon la revendication 3, dans laquelle le peptide présente la séquence d'acides aminés suivante :
Gly-Pro-Arg-Pro-Lys.

5. Utilisation selon la revendication 1, **caractérisée en ce que** le ligand est un anticorps.

6. Utilisation selon l'une des revendications 1 à 5, **caractérisée en ce que** le mammifère est un être humain.

7. Utilisation selon l'une des revendications 1 à 6, **caractérisée en ce que** le ligand est choisi parmi des anticorps anti-fibrinogène et des anticorps anti-fibrine polyclonaux et monoclonaux.

8. Utilisation selon l'une des revendications 1 à 7, **caractérisée en ce que** le ligand est lié à une matrice solide dans l'agent.

9. Utilisation selon la revendication 8, **caractérisée en ce que** la matrice est choisie parmi le verre, des hydrates de carbone, des polyméthacrylates et des polyamides.

10. Utilisation selon la revendication 9, **caractérisée en ce que** la matrice est le sépharose.

11. Utilisation selon l'une des revendications 8 à 10, **caractérisée en ce que** la matrice se compose de billes, de fibres et/ou d'une membrane.

12. Utilisation selon l'une ou plusieurs des revendications précédentes, **caractérisée en ce que** le trouble de la microcirculation se manifeste en relation avec le diabète, la rétinopathie, la polyneuropathie, l'apoplexie, la perte brutale de l'audition, le sepsis, des maladies artérielles occlusives et/ou une compromission de la fonction rénale.

13. Colonne adsorbante contenant une matrice et un ligand, le ligand possédant une spécificité pour la fibrine et/ou pour le fibrinogène.

14. Colonne adsorbante selon la revendication 13, dans laquelle le ligand est le peptide indiqué dans l'une des revendications 3 ou 4.

15. Colonne adsorbante selon la revendication 13 ou 14, dans laquelle la matrice est le sépharose.

16. Procédé permettant d'agir sur la microcirculation d'un mammifère, dans lequel le sang *in vitro* du mammifère passe dans la colonne selon la revendication 13, 14 ou 15.

17. Procédé selon la revendication 16, **caractérisé en ce qu'**il s'effectue sous la forme d'un procédé d'aphérèse dans le plasma ou le sang total.
